# EUROPEAN PATENT APPLICATION

(11) **EP 1 686 131 A2**
(43) Date of publication of application: **02.08.2006**
(21) Application number: 06001654.0
(22) Date of filing: 26.01.2006
(51) Int. Cl.: C07D 499/08

(54) **Process for preparation of penam derivatives**

(30) Priority: 28.01.2005 US 46080
(71) Applicant: Helm AG, 20097 Hamburg (DE)
(72) Inventor: Gegö, Csaba Lehel, 1181 Budapest (HU); Fejes, Imre, 1012 Budapest (HU); Kovács, Imre, 4034 Debrecen (HU); Lukács, Ferenc, 2143 Kistarcsa (HU); Schneider, Géza, 1097 Budapest (HU)
(74) Representative: Maiwald Patentanwalts GmbH

(57) **Abstract**

The invention relates to novel processes for preparing penam derivatives, such as Tazobactam and derivatives thereof. The processes according to the invention encompass procedures for the protection and deprotection of the carboxylic group as well as for the oxidation of the sulphur moiety of penam derivatives. Additionally, the present invention relates to new intermediates for the production of penam derivatives, allowing the desired penam-derivatives to be formulated with high purity and in good yields.

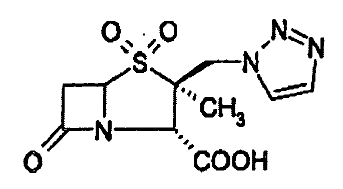

## Description

### Technical field

The present invention relates to a process for preparing penam derivatives. More particularly the present invention provides a novel process for preparing Tazobactam and derivatives thereof. 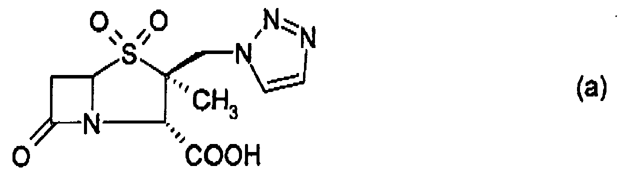 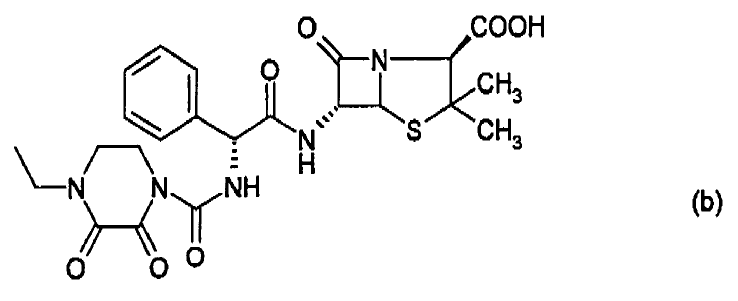 A mixture of Tazobactam (a) and Piperacillin (b) as sodium salts has utility as an antibacterial.

### Background of the invention

The usage of β-lactam antibiotics like Piperacillin is limited by the resistance exhibited by the micro-organisms through the action of the β-lactamase enzyme. The enzyme acts through cleavage of the β-lactam ring of these antibiotics, thereby destroying the drug and leading to loss of activity. Therefore β-lactamase inhibitors like Tazobactam are useful, as they counteract the β-lactamase enzyme and eliminate drug resistance. The β-lactamase inhibitors are used along with β-lactam antibiotics to promote the antibiotic activity. Thus research on new penam derivatives and novel processes for their production are of value.

### Description of prior art

Several patents disclose various methods of producing β-substituted methyl penam derivatives. For instance, US 4,529,592 discloses a process which involves the treatment of 2α-methyl-2β-azidomethyl penam derivatives of formula (c): 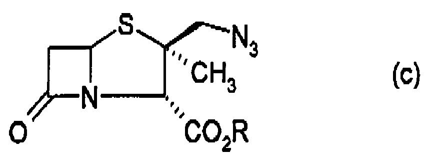 wherein R is a carboxy-protecting group, with acetylene, an acetylene derivative or a vinyl derivative under high pressure in a sealed reactor and at elevated temperatures, followed by deprotection with a suitable reagent to get the β-lactamase inhibitor of formula (a).

The 2α-methyl-2β-azidomethyl penam derivative of formula (c) is in turn prepared from the 2α-methyl-2β-halomethyl penam derivatives of formula (d) 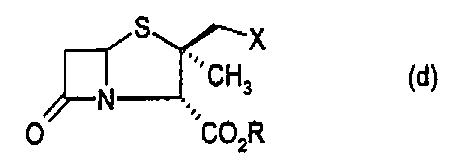 wherein R is a carboxy-protecting group and X is chloro or bromo, by treating with sodium azide in aqueous polar aprotic solvents, followed by oxidation.

US 4,891,369 and US 4,933,444 disclose a different approach, which involves the preparation of 2α-methyl-2β-triazolylmethylpenam derivatives of formula (e) wherein R is a carboxy protecting group and n is 0, by the treatment of a β-halomethyl penam derivative of formula (d), wherein X is chlorine or bromine and R is a carboxy-protecting group, with 1H-1,2,3-triazole. 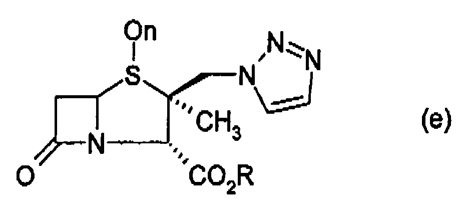 The product obtained can be oxidized and deprotected to get the 2β-substituted methyl penam compound (a).

US 4,912,213 discloses a reduction method employing lead salts in catalytic amounts to prepare a 2α-methyl-2β-triazolylmethyl penam derivative of formula (e) (n=0-2) from 6-halo or 6,6-dihalo-2α-methyl-2β-triazolylmethyl penam derivatives of formula (f) 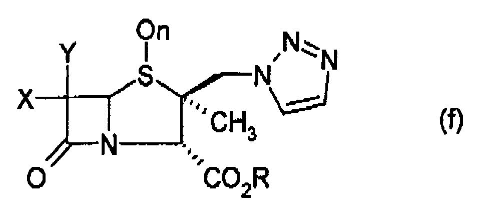 where X may be Cl, Br, I; Y may be Cl, Br, I or a hydrogen atom; and R is a carboxy-protecting group.

In yet another method disclosed by US 4,895,941, penam sulfoxide of formula (g), wherein R represents a carboxy-protecting group, is treated with 2-trimethylsilyl-1,2,3-triazole in a sealed tube at elevated temperatures to give a mixture which requires purification by column chromatography to isolate the 2α-methyl-2β-triazolylmethyl penam derivative of formula (e) (n=0).

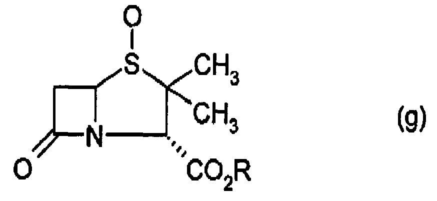

As an alternative to the hydrogenation, US 4,925,934 discloses a deblocking method for a 2α-methyl-2β-triazolylmethyl penam derivative of formula (h) by reaction with cresol 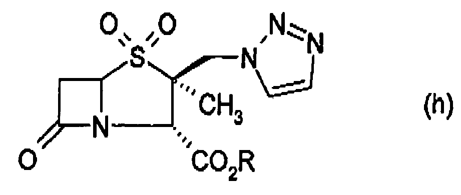 where R is selected from p-methoxybenzyl, 3,4,5-trimethoxybenzyl, 2,4-dimethoxybenzyl, 3,5-dimethoxy-4-hydroxybenzyl, 2,4,6-trimethylbenzyl, diphenylmethyl, ditolylmethyl, dianisylmethyl or tert-butyl.

Published application US 2003/232983 discloses a complete different route of synthesis for 2α-methyl-2β-triazolylmethyl-penam derivatives starting from cepham derivates of formula (i) by substitution and rearrangement 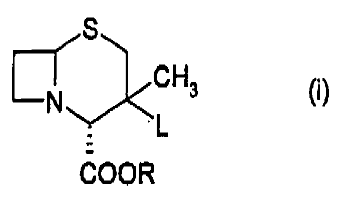 where R represents a carboxy-protecting group and L a leaving group.

In most of the methods involved, 2α-methyl-2β-halomethyl penam of formula (d) is used as the key intermediate. This is true with both the azide/acetylene combo and the triazole route discussed above. However, the 2α-methyl-2β-halomethyl penam of formula (d) itself is an unstable intermediate and therefore manufacturing and storage of this intermediate in large quantities is always cumbersome. This intermediate has been found to degrade on storage even at low temperatures in isolated form as well as in the solution from which it is isolated. Thus, all the operations related to preparation of the intermediate have to be done rapidly, and the isolated intermediate has to be converted to the final product immediately. As a result of these limitations, in-plant scale up always yields by-products which ultimately require purification demands.

Furthermore, the deblocking of 2α-methyl-2β-triazolyl penam derivatives to Tazobactam by hydrogenation has the crucial drawback of catalyst poisoning and therefore a lower yield and purity. The hydrolysis with cresol overcomes this drawback but introduces a very toxic reagent at the last stage of the synthesis route.

All the above described processes are associated with one or more of the following limitations: (i) unstable nature of the key intermediate; (ii) expensive purification by column chromatography; and (iii) use of highly toxic compounds like lead or cresol, especially in the penultimate stages of pharmaceuticals. These factors affect consistency in quality and yield of both the intermediates and the final product, and can impact upon pharmaceutical usage consistent with regulatory requirements.

In accordance with the foregoing, it is a purpose of the present invention to provide a new and improved method for the manufacture of Tazobactam and related compounds. A further purpose of the invention is to provide such a method that yields Taczobactam and such related compounds of high purity and high yield.

### Brief description of the invention

The present invention relates to a novel process for 2α-methyl-2β-triazolyl penam derivatives in high yield and high purity. In particular, this invention concerns a novel process for preparing a 2α-methyl-2β-triazolyl penam derivative represented by formula (j) 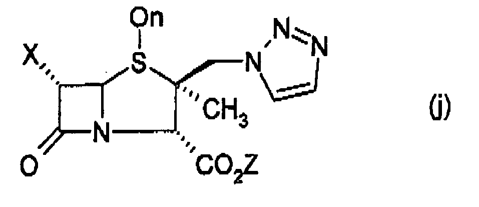 wherein X is hydrogen or bromine; n is 0,1 or 2; and Z is para-nitrobenzyl, hydrogen, sodium or a quaternary amomnium of the type NR₄ wherein R is a substituted or unsubstituted, linear or branched alkyl chain like NBu₄ as a non-limiting example.

Debromination of a starting compound is followed by deblocking by catalytic hydrogenolysis or hydrolysis. The resulting compound is reacted with a quaternary amomnium salt. Ion exchange is applied to yield the desired derivative.

Alternate reaction pathways may be employed for carrying out the process of the invention. As used herein, three of such alternative pathways are denoted as routes A, B, and C, and illustrative examples of the invention utilizing such pathways are presented.

### Brief description of the drawings

The invention may be more fully understood with reference to the following detailed description of an illustrative embodiment thereof when considered in connection with the annexed drawings, in which:

Figure I is a reaction flow sheet of the method of the invention incorporating a first synthesis route (route A);

Figure II is a reaction flow sheet of the method incorporating a second, alternate synthesis route (route B); and

Figure III is a reaction flow sheet of the method incorporating a third alternate synthesis route (route C).

### Detailed Description of the invention

The present invention provides a process for the preparation of Tazobactam represented by formula (a) and/or pharmacological acceptable salts thereof. 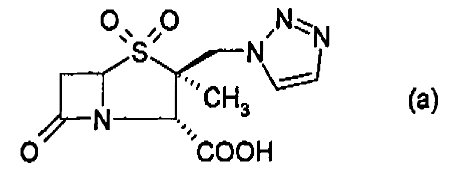 The process is represented by the following reaction steps consisting of debromination of compound (k) 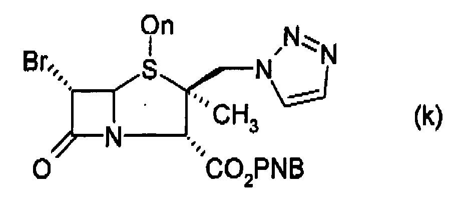 wherein PNB is para-nitrobenzyl, n = 0, 1 or 2 to give compound (1) 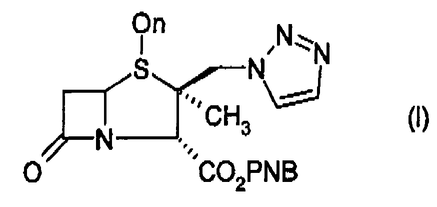 wherein n is as defined above, further comprising the deblocking of compound (I) to give compound (m) either by catalytic hydrogenolysis or hydrolysis with a hydrogensulfide salt like sodium hydrogensulfide, optionally in the presence of an alkali or earth alkali metal salt, 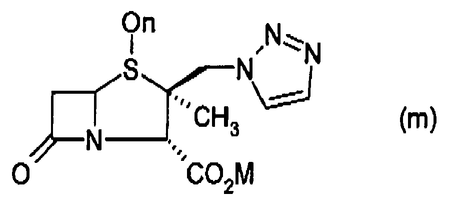 wherein n is as defined above and M is hydrogen or an alkaline or alkaline earth metal, such as sodium. The process further comprises reacting compound (m) with a quaternary amomnium salt NR₄Y wherein R is defined above, comprising in the case of n<2 that Y is an anion with oxidising capabilities like HSO₅ to yield compound (n), which can be purified by extraction, 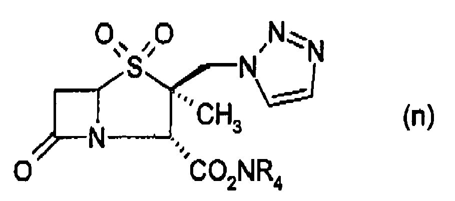 wherein R is defined above. Finally the process comprises the ion exchange of the NR₄ of compound (n) salt to yield compound (a) or a pharmacologically acceptable salt thereof .

Utilization of the PNB-protection group for the acid moiety in penam derivatives and the removal of these or related benzyl-protection-groups with hydrogen sulfide salts avoids the necessity of utilizing toxic and undesirable compounds like cresol or metal catalysts but leads to unexpected high yields, which were not achievable from other deblocking-methods known from the state of the art. Utilization of quaternary ammonium salts of the penam compounds allows for easy and very advantageous purification of this intermediate and makes the desired penam-product available in high purity and good yields. Additionally, introduction of the quaternary ammonium group provides an opportunity for generating the sulfonyl compound (n) from the corresponding thioether or sulfinyl derivative by a simple reaction with a quaternary ammonium salt possessing a counter-anion with oxidation capabilities. Removal of the quaternary ammonium group leading to the free penam-acid or the corresponding pharmaceutical acceptable salts is achievable by simple procedures, e.g. ion exchange.

The methodology of deblocking para-nitrobenzyl esters of penam derivatives with hydrogen sulphide and using quaternary amomnium salts of Tazobactam for the purification by extraction is not known in the literature and in the light of prior art it involves an inventive step for a person skilled in the art, therefore the whole invention is new and inventive.

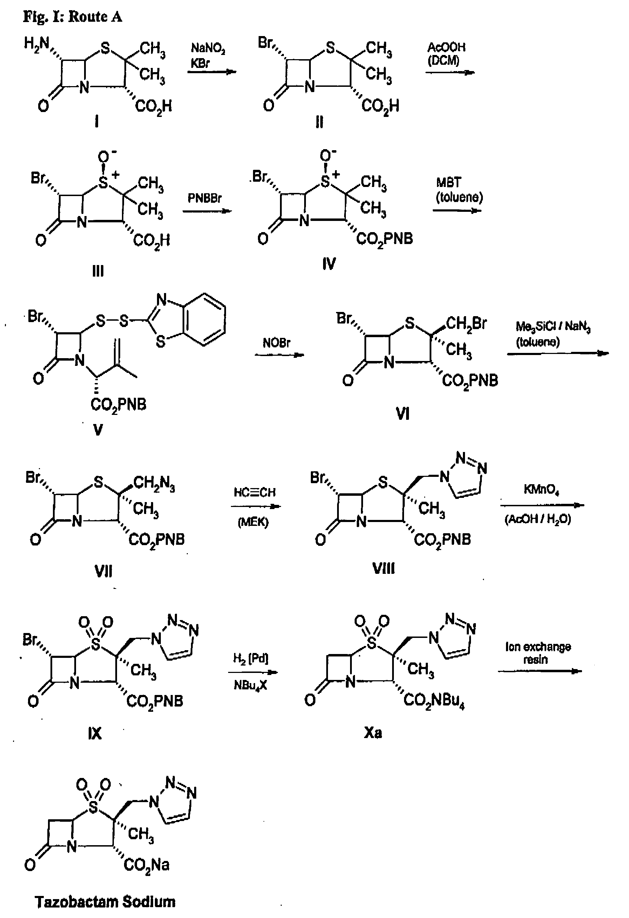

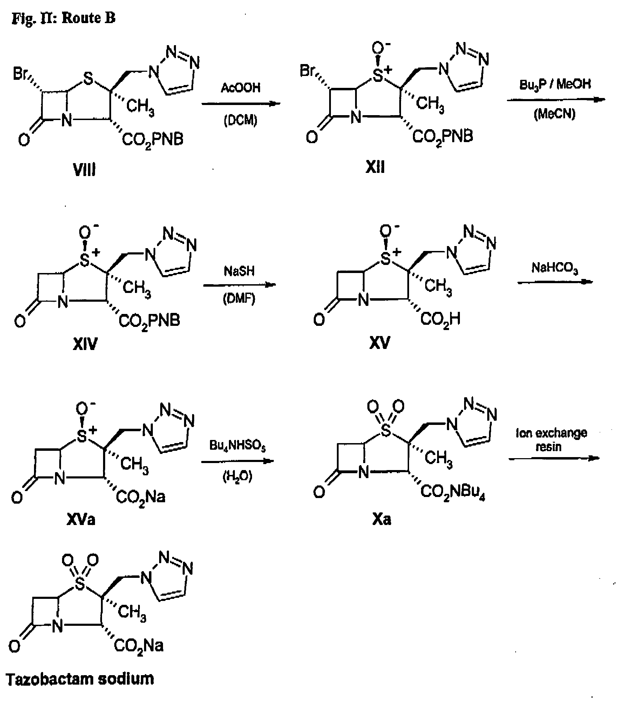

### Example 1: Preparation of Tazobactam Sodium by route A. (Fig. 1)

### Step 1. Production of 6α-Bromopenicillanic acid (BPA) (compound II)

2.5 L of 1.24 molar sulphuric acid (3.125 mol) was stirred at 4°C in a 6 L flask. 218.4 g (1.0 mol) of 6-APA (99%) (compound I) following 601 g (5.05 mol) of potassium bromide and 2000 mL of ethanol were added, maintaining the temperature between 4 to 8°C. Inorganic salts were removed by filtration. The resulting cake was washed by 2 x 1.25 L of cooled dichloromethane. The aqueous phase was extracted twice using the previous washing liquor and 3 x 500 mL of cooled dichloromethane. The organic phases were combined (approx. 4.0 L) and washed with 2 x 200 mL of 30% brine at 4°C. The greenish-brown solution was concentrated to 700 mL in vacuum. The precipitate was removed by filtration and the solution was kept below 0°C and used without further purification in the next reaction step.
Yield: 90% (by titration)
TLC (thin layer chromatography; detection by UV and phosphomolybdic acid, eluent: acetone - methanol 2:1 v/v): R_{f} 0.65 (BPA), (eluent: acetone - methanol 4:1 v/v) R_{f} 0.35 (BPA)

### Step 2. Production of 6α-Bromopenicillanic acid-S-oxide (BPO) (compound III)

1.8 mol of BPA in 1400 mL of dichloromethane was placed in a 4 L flask. The temperature of the solution was maintained between 0 to 2°C. 2.0 mol peracetic acid in acetic acid solution (342 mL, 40 wt.-% peracetic acid) was added within 100 to 120 minutes, maintaining the temperature of the solution between 0 to 8°C. The color of the solution changed to yellowish-brown. The solution was stirred further 1 hour at 0 to 8°C. The product crystallizes. The slurry was cooled to -10 to -15°C and stirred further 30 minutes then filtered. The cake was washed with 2 x 400 mL of dichloromethane at -10°C. The product was dried at 20 - 25°C in vacuum. The crude product was kept below 0°C and used without further purification immediately (storage time 1 to 2 days) in the next reaction step.
Yield: 314 - 331g (58,9 - 62.1 %) Mp: 130 °C (decomp.)
Cumulative yield of 1^{st} and 2^{nd} steps: 51- 52%
TLC (detection by UV and phosphornolybdic acid, eluent: acetone - methanol 2:1 v/v)
R_{f} 0.65 (BPA), R_{f} 0.45 (BPO)
The yield can be improved using higher concentrated peracetic acid.

### Step 3. Production of 6α-Bromopenicillanic acid-S-oxide p-nitrobenzyl ester (BPE) (compound IV)

In a 4 L flask 272.44 g (0.92 mol) of BPO was dissolved in 120 mL DMF at 25°C. 100.8 g (1,2 mol) of sodium hydrogencarbonate and 229.0 g (1.06 mol) of p-nitrobenzylbromide (PNM) were added portionwise. The slurry was cooled and stirred at 0 to 5°C for one hour. The product was filtered and washed with 2 x 800 mL of cold water. The wet product was placed in a 2 L flask and 1200 mL of methanol was added. The slurry was refluxed for one hour, cooled to -10°C and filtered. The cake was washed with 2 x 800 mL of methanol at -10°C. The product was dried at 25 - 30°C in vacuum and stored at 0°C without further purification in the next reaction step.
Yield: 334.8 g (84.4%) Mp: 130 °C (decomp.)
Cumulative yield of 1^{st}, 2^{nd} and 3^{rd} steps: 46%
TLC (detection by UV, eluent: acetone - methanol 2:1 v/v) R_{f} 0.75 (BPE), R_{f} 0.65 (BPO);
(eluent: ethyl acetate - hexane 2:1 v/v) R_{f} 0.50 (BPE), R_{f} 0.00 (BPO)

### Step 4. Production of 2-(2-Benzothiazolyldithio)-3-bromo-α-(1-methylethylidene)-4-oxo-1-azetidincacetic acid p-nitrobenzyl ester (BBE) (compound V)

In a 4 L flask 140.84 g (0.826 mol) of 95% 2-mercaptobenzothiazole (MBT) and 345.0 g BPE (0.8 mol) were dissolved in 1360 mL toluene when the solution was heated to 86 - 90°C and an azeotropic mixture of toluene-water was distilled at 450 to 500 mbar. After 3 to four hours, 14 to 16 mL of water was removed using a Dean-Stark apparatus maintaining the temperature between 86 to 90°C. If unreacted BPE could be detected by TLC, a small amount of 2 to 8 g of MBT was added. The solution was refluxed until no starting material could be detected by TLC.

The solution was evaporated in vacuum between 60 to 70°C. The residual oil was dissolved in 1200 mL of ethyl acetate. After cooling the product crystallizes. The slurry was concentrated in vacuum below 50 °C to 800 mL and 1200 mL isopropyl ether was added to give a well-filterable crystalline slurry that was cooled below 20°C and stirred for additional 24 hours. Subsequently, the product was filtered and washed with 2 x 500 mL cooled isopropyl ether. The product was dried in vacuum between 25 - 30°C.
Yield: 412.8 g (88.9%) Mp.: 116-119°C
Cumulative yield of 1^{st}, 2^{nd}, 3^{rd} and 4^{th} steps: 41%
TLC (detection by UV, eluent: isopropyl ether - ethyl acetate 99:1 v/v) Rᵣ 0.65 (BBE)

### Step 5. Production of 6α-Bromo-2β-bromomethyl-2α-methylpenam-3α carboxylic acid p-nitrobenzyl ester (DBPE) (compound VI)

In a 4 L flask 290.24 g (0.5 L) of BBE was dissolved in 1500 mL dichloromethane. The solution was cooled to -2°C. 540 mL of 30% aqueous solution of hydrogen bromide (2.52 mol) was added, keeping the temperature below 0°C. A solution of 103.5 g (1.5 mol) sodium nitrite in 300 mL was added keeping the temperature between 0 to 3 °C. Meanwhile the colour of the organic phase turned to brown. The reaction mixture was stirred about 90 min at 0 to 5 °C until the starting material could not be detected by TLC. 80 g of sodium carbonate (0.75 mol) was added, adjusting the pH to between 6 and 7. The reaction mixture was filtered using perlite as a filter aid. The precipitate was washed with 3 x 100 mL dichloromethane. The combined organic layer was separated and concentrated to 700 mL. The solution was cooled to 20 °C and two litres of isopropyl ether were added slowly. The crystalline suspension was stirred 16 hours at 20 °C and two hours at 0 °C. It was filtered and the product was washed with 2 x 300 mL of cooled isopropyl ether. The product was dried at 20 to 25 °C in vacuum.
Yield: 235.84 g (95.5%) Mp.: 80 °C (decomp.)
Purity: min. 95 %
Cumulative yield of 1^{st} - 5^{th} steps: 39%
The product is sensitive to light and decomposes on silica gel to give cepham.
TLC (detection by UV, eluent: isopropyl ether - ethyl acetate 99:1 v/v) R_{f} 0.72 (DBPE),
R_{f} 0.65 (BBE), R_{f} 0.57 (cepham)

### Step 6. Production of 6α-Bromo-2β-azidomethyl-2α-methylpenam-3α-carboxylic acid p-nitrobenzyl ester (BTPE) (compound VII)

In a 2 L flask 292.3 g (342 mL, 2.664 mol) trimethylsilylchloride was dissolved in 1300 mL of toluene. 210.1 g (3.20 mol) sodium azide was added and the suspension was stirred and refluxed. The reaction was traced by GC. After 10 to 16 hours less than 0.1% of the starting material could be detected. The suspension was cooled to -5 to 0°C and was filtered (or decanted). The solution (1580 mL) contains 2.40 mol of trimethylsilylazide, which is volatile (Bp: 95°C) and a toxic compound.

In a 2 L flask 52.63 g (23.7 mL, 0.2 mol) tin(IV) chloride was added to a toluene solution of 2.4 mol of trimethylsilylazide between 20 - 25°C. The solution was stirred 24 hours at 20 - 25 °C while some white precipitate appeared. 197.7 g (0.4 mol) DBPE was added. The suspension was stirred 40 to 70 hours while brown gum appeared. The formation of azide was traced by TLC (eluent isopropyl ether - ethyl acetate 99:1 v/v) R_{f} 0.72 (DBPE), R_{f} 0.61 (BAPE), R_{f} 0.58 (cephambromide) R_{f} 0.40 (cephamazide).

Conversion of the starting material to product was less than 50% after 40 hours. Additionally, 0.2 mol of tin (IV) chloride was added, which accelerated the formation of BAPE.

After no starting material could be detected by TLC, the reaction mixture was quenched with 1200 mL of saturated sodium carbonate solution at 5-10°C. The insoluble material was dissolved by 400 mL ethyl acetate and added to the sodium carbonate solution. The biphasic reaction mixture was stirred 15 minutes, The pH of the lower aqueous phase was between 8 and 9. Perlite (50 g) as a filter aid was added and the suspension was filtered. The cake was washed with 2 x 200 mL of ethyl acetate.

The combined filtrates were poured into a 5 L separating funnel and the lower aqueous phase was removed and extracted with 2 x 200 mL ethyl acetate. The combined organic phases were washed by 200 mL saturated sodium bicarbonate solution and 200 mL brine. The solvent was removed in vacuum and the residue was suspended in 1000 mL methanol at 0 - 5 °C. The crystalline suspension was stirred 2 to 3 hours at 0 - 5 °C and filtered. The product was washed with 200 mL diisopropyl ether and dried in vacuum at 20 - 25 °C.
Yield: 153.8 g (84.3%)
Purity: 68 ― 70% (by HPLC: mobile phase 0.05 M KH₂PO₄ - acetonitrile 1:1, pH 6,
R_{f} 14.33 min)
Cumulative yield of 1^{st} - 6^{th} steps: 33%

### Step 7. Production of 6α-Bromo-2β-[(1,2,3-triazol-1-yl)methyl]-2α-methylpenam-3α-carboxylic acid p-nitrobenzyl ester (BTPE) (compound VIII)

In a 1 L autoclave 7.6 g (50 mmol) BAPE was dissolved in 640 mL 2-butanone. The solution was cooled down to 0 - 5 °C. The autoclave was pressured three times with nitrogen gas up to six bar. The autoclave was filled with acetylene gas up to 1.5 bar pressure and approx. 36 g acetylene gas was dissolved. The autoclave was heated gradually from 0 °C up to 84 - 94 °C, keeping the pressure between 5 - 6 bar. The reaction mixture was stirred in the autoclave 14 - 20 hours at 84 to 94 °C and pressure of 5 to 6 bar. No starting material was detected by TLC (eluent hexane - ethyl acetate 1:2 v/v) R_{f}> 0.9 (BAPE), R_{f} 0.51 (BTPE), R_{f} 0.32 (cephamtriazole).

The autoclave was cooled down to -20 to -25 °C and 7.6 g BAPE in 50 mL 2-butanone solution was added. The autoclave was heated again to 84 - 94 °C and the reaction mixture was stirred 14 to 20 hours at 84 - 94 °C. The autoclave was cooled and the procedure was repeated with 7.6 g BAPE. The autoclave was cooled down to 20 - 25 °C and opened. The reaction mixture was poured into a 1 L flask and was concentrated in vacuum up to 140 mL. The solution was cooled to 0 - 5°C. The crystalline suspension was stirred for 1 hour and was filtered. The product was washed with 40 mL cool 2-butanone. The product was dried in vacuum at 25 - 30 °C.
Yield: 13.51 g (56.0%) Mp.: 180-182°C (decomp.)
Purity: 98.6% (by HPLC: mobile phase 0.05 M KH₂PO₄ - acetonitrile 1:1, pH 6,
R_{f} 8.40 min)
Cumulative yield of 1^{st}- 7^{th} steps: 18%

### Step 8. Production of p-Nitrobenzyl 6α-bromo-2α-methyl-2β-(1,2,3-triazol-1-yl)methylpenam-3α -carboxylate-1,1-dioxide (compound. IX)

To a solution of 4.82 g (10.00 mmol) of BTPE in a mixture of 210 ml of acetic acid and 27 ml of water, 3.79 g (23.6 mmol) of KMnO₄ was added in 30 minutes at room temperature. The progress of the reaction was monitored by TLC. When the reaction was complete, the excess of KMnO₄ was destroyed by 30 % H₂O₂ solution. The reaction mixture was poured into 930 mL of cold water, the precipitated product was filtered and washed with cold water and dried over P₂O₅, giving compound IX.
Yield: 4,12 g (80 %)
Purity: more than 95 % (HPLC) Mp.: 122-124°C
TLC (detection by UV, eluent: ethyl acetate - hexane 2:1 v/v) R_{f} 0.51 (VIII), R_{f} 0.23 (IX)

### Step 9. Production of Tetrabutylammonium 2α-methyl-2β-(1,2,3-triazol-1-yl)methylpenam-3α -carboxylate-1,1-dioxide (compound Xa)

A stainless steel stirred autoclave with a total volume of 1 L was charged with 5.1 g (10 mmol) of compound IX, 2.5 g (30 mmol) of NaHCO₃, 1.0 g of 10 % Pd on charcoal, 100 mL of water and 100 mL of ethyl acetate. The autoclave was sealed and flushed with argon, then pressured with hydrogen up to 14 bars. The hydrogenation was carried out at room temperature for 5 h. Completion of the reaction was checked by TLC. The mixture was filtered and the filter washed with water. The aqueous phase was separated, washed with ethyl acetate (2 × 10 mL) and Bu₄NNaSO₄ solution (prepared from 340 mg (1 mmol) of Bu₄NHSO₄ and 84 mg (1 mmol) of NaHCO₃ in 5 mL of water) added. The aqueous solution was extracted with dichloromethane (5 x 10 ml). The combined dichloromethane phases were dried over Na₂SO₄ and concentrated under reduced pressure to dryness keeping the temperature of the water bath below 20 °C.
Yield: 0.39 g (75 %)
Purity: 95.5 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.53 min
Column: RP-18 endcapped (5µm, 250 mm)
TLC (detection by UV and 1 % AgNO₃ in ethanolic solution, eluent: ethyl acetate - hexane 2:1 v/v) R_{f} 0.23 (IX); (eluent: acetone -methanol 2:1 v/v) R_{f} 0.48 (Xa)

### Step 10. Production of Sodium 2α-methyl-2β-(1,2,3-triazol-1-yl)methylpenam-3α-carboxylate-1,1-dioxide (Tazobactam sodium)

The residue containing compound Xa (0.40 g) was eluted with water on a column of Amberlite-Na⁺ cation-exchange resin. The appropriate fractions were concentrated under reduced pressure and finally lyophilized, yielding Tazobactam sodium.
Yield: 0.21 g (85 %)
Purity: 99.5 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.53 min
Column: RP-18 cndcapped (5µm, 250 mm)

### Example 2: Preparation of Tazobactam Sodium by route C. (Fig. 3)

### Step 1. BTPE (compound VIII) is produced, such as through steps 1 - 7 of Example 1.

### Step 2. Production of p-Nitrobenzyl 2α-methyl-2β-[(1,2,3-triazol-1-yl)methyl]-penam-3α-carboxylate (compound XI)

A solution of 300 mL acetonitrile, 50 mL methanol 24.10 g of compound VIII and 13 mL tributylphosphine was prepared. After 4 hours of stirring, remains of the starting material could be detected by TLC (eluent: hexane/ethyl acetate 1:2 v:v; R_{f} (VIII) = 0,60; R_{f} (Tazo-XII) = 0.45). Then, 3 mL of tributylphosphine was added and the mixture was stirred for further 2 h. The solvent was removed *in vacuo* at 40°C. The oily residue was dissolved in 80 mL methanol and cooled down to -20 °C. After 12 hours, the precipitate was filtered and washed with 20 mL methanol and 20 mL isopropyl ether at -20°C. Finally, the product was dried in vacuum.
Yield: 17.08 g (84.7 %) Mp.: 109 - 114 °C
Purity: 99.41% (by HPLC)

### Step 3. Production of 2α-Methyl-2β-(1,2,3-triazol-1-yl)methylpenam-3α-carboxylic acid (compound XIII)

In a 100-mL two-necked round-bottomed flask, equipped with magnetic stirring bar and thermometer 8.06 g (20 mmol) of compound XI was dissolved in 70 mL of dimethylformamide. The stirred solution was cooled with an ice/water bath, then under argon atmosphere 2.96 g (40 mmol) of NaHS^{.}H₂O was added portionwise over a period of 2 h while the temperature was maintained below 10 °C. Completion of the reaction was checked by TLC. After the addition, the dark solution is poured into 7 mL of acetone and the precipitated solid is filtered then washed with acetone (2 x 2 mL). The yellow colored sodium salt was dried *in vacuo* over P₂O₅ at room temperature.
Yield: 4.90 g (84 %)
Purity: 85.1 % (HPLC)

The sodium salt was dissolved in 0.5 mL of sat. NH₄Cl solution then acidified to pH 2 with 10 % aq. H₂SO₄. The precipitate was filtered and washed with water (2 x 1 mL) then dried *in vacuo* over P₂O₅ at room temperature.
Yield: 2.5 g (47 %)
Purity: 99.1 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 13.81 min
Column: RP-18 endcapped (5µm, 250 mm)
TLC (detection by UV and 1 % AgNO₃ in ethanolic solution, eluent: ethyl acetate - hexane 2:1 v/v) R_{f} 0.28 (XI); (eluent: acetone -methanol 3:1 v/v) R_{f} 0.18 (XIII)

### Step 4. Production of Tetrabutylammonium 2α-methyl-2β-(1,2,3-triazol-1-yl)methylpenam-3α-carboxylate-1,1-dioxide (compound Xa)

To a cold solution of 0.29 g (1.00 mmol) of compound XIIIa (as may be prepared from compound XIII and NaHCO₃) and 0.176 g (2.1 mmol) NaHCO₃, 0.745 g (2.10 mmol) tetrabutylammonium hydrogenpersulfate was added in small portions with vigorous stirring such that 5°C was not exceeded. The reaction mixture was stirred further 24 hours at between 0-5°C and extracted with 5 x 10 ml of dichloromethane. The combined organic layers were washed with 5 ml of water and evaporated to dryness keeping the temperature of the water bath below 20°C, yielding compound Xa.
Yield: 0.40 g (77 %)
Purity: 98.0 % (HPLC)
HPLC mobile phase; 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.34 min
Column: RP-18 endcapped (5µm, 250 mm)
TLC (detection by UV eluent: acetone -methanol 2:1 v/v) R_{f} 0.48 (XIIIa); R_{f} 0.48 (Xa)

### Step 5. Production of Sodium 2α-methyl-2β-(1,2,3-triazol-1-yl)methylpenam-3α-carboxylate-1,1-dioxide (Tazobactam sodium)

As in Example 1, The residue containing compound Xa (0.40 g) was eluted with water on a column of Amberlite-Na⁺ cation-exchange resin. The appropriate fractions were concentrated under reduced pressure and finally lyophilized, yielding Tazobactam sodium.
Yield: 0.20 g (85 %)
Purity: 99.5 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.53 min
Column: RP-18 endcapped (5µm, 250 mm)

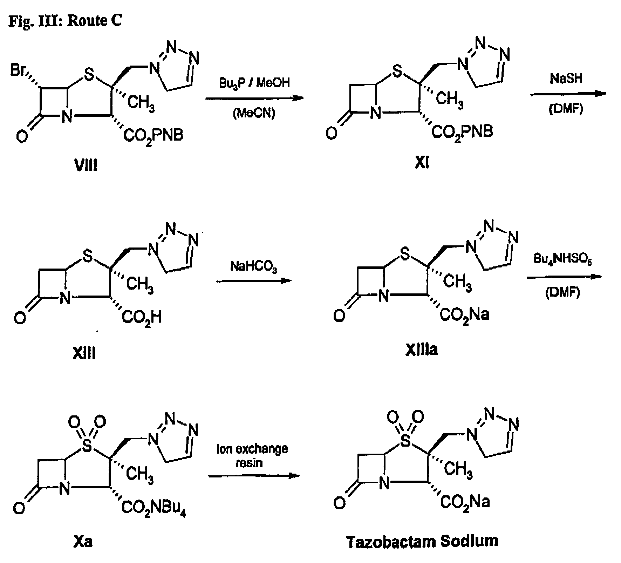

### Example 3: Preparation of Tazobactam Sodium by route B. (Fig. 2)

### Step 1. BTPE (compound VIII) is produced, such as through steps 1 - 7 of Example 1.

### Step 2. Production of p-Nitrobenzyl 6α-Bromo-2α-methyl-2β-[(1,2,3-triazol-1 yl)methyl]-penam-3α-carboxylate-1α-oxide (compound XII)

Compound VIII (16.88 g) was dissolved in 350 mL ofdichloromethane. The mixture was cooled to 6°C and 10 mL of peracetic acid (38-40%; ca. 58 mmol) was added. The mixture was stirred for 3.5 hours and kept between 2 - 7 °C until no educt could be detected in the TLC (eluent: hexane/ethyl acetate 1:2; R_{f} (VIII): 0.60, R_{f} {XIV): 0.23). The solution was washed 2x with 100 mL of water, 100 mL of aqueous saturated sodium bicarbonate solution and then again with 100 mL of water. The solution was concentrated to 30 mL and the product started to crystallize. 70 mL of methanol was added to the suspension and the remaining dichloromethane was distilled from the solution. The mixture was stirred for 1 hour at 2 - 6°C. After filtration and washing with cold methanol, the product was dried *in vacuo.*
Yield: 16.3G g (93.8 %) Mp.: >150 °C
Purity: 96.8% (by HPLC)

### Step 3. Production of p-Nitrobenzyl 2α-methyl-2β-[(1,2,3-triazol-1-yl)methyl]-penam-3α-carbaxylate-1α-oxide (compound XIV)

To a stirred suspension of XII (19.85 g) in a mixture of 200 mL of dichloromethane and 15 mL of methanol, 10.4 mL (42.00 mmol) of tributylphosphine was added over 30 min at 0°C. After completion of the addition a clear solution was obtained. The progress of the reaction was monitored by TLC. When no more starting material was detectable (after about 2 h) the reaction mixture was diluted with 100 mL of methanol and concentrated to 100-110 mL in vacuum. The obtained suspension was stirred at 0-5°C for 1 h. The precipitated crystals were collected by filtration, washed with 15 mL of -10°C methanol and dried in vacuum at room temperature.
Yield: 13.3-14.7 g (80-88%) Mp.: 99-103 °C
Purity: 98.9 % (HPLC)
TLC (detection by UV, eluent: ethyl acetate - hexane 2:1 v/v) R_{f} 0.23 (XIV), R_{f} 0.09 (XV)

### Step 4. Production of 2α-Methyl-2β-(1,2,3-triazol-1-yl)methylpenam-3α-carboxylic acid-1α-oxide (compound XV)

In a 50-mL, two-necked, round-bottomed flask, equipped with magnetic stirring bar and thermometer 2.94 g (7 mmol) of compound XIV was dissolved in 20 mL of dimethylformamide. The stirred solution was cooled with an ice/water bath, then under an argon atmosphere 1.04 g (14 mmol) of NaHS^{.}H₂O was added portionwise over a period of 2 h while the temperature is maintained under 10 °C and completion of the reaction was checked by TLC. After the addition, the dark solution was poured into 100 mL of acetone and the precipitated solid filtered, then washed with acetone (2 x 2 mL). The orange coloured sodium salt was dried *in vacuo* over P₂O₅ at room temperature.
Yield: 1,95 g (91 %)
Purity: 82.5 % (HPLC)

The sodium salt was dissolved in 0.5 mL of sat. NH₄Cl solution then acidified to pH 2 with 10 % aq. H₂SO₄. The precipitate is filtered and washed with water (2 x 1 mL) then dried in vacuo over P₂O₅ at room temperature.
Yield: 1.21 g (60 %)
Purity: 99.1 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 4.87 min
Column: RP-18 endcapped (5µm, 250 mm)
TLC (detection by UV and 1% AgNO₃ in ethanolic solution; eluent: ethyl acetate - hexane 5:1 v/v) R_{f} 0.10 (XIV); (eluent: acetone - methanol 3:1 v/v) R_{f} 0.13 (XV)

Alternate production of 2α-Methyl-2β-(1,2,3-triazol-1-yl)methylpenam-3α-carboxylic acid-1α-oxide sodium salt (XVa)
In a 250 mL, one-necked, round-bottomed flask, equipped with magnetic stirring bar 7.50 g (17.9 mmol) of XIV is dissolved in 150 ml of tetrahydrofuran / methanol (1/1) mixture then 375 mg of 10 % Pd on charcoal is added. The flask is closed with a rubber septum and the vigorously stirred solution is washed through with argon then hydrogen. The hydrogenation is continued under atmospheric pressure for ca. 20 hours (completion of the reaction is checked by TLC). The mixture is evaporated, then 40 ml of THF, 40 mL of ethyl acetate, 40 mL, of water and 1.50 g (17.9 mmol) of NaHCO₃ are added. The mixture is stirred vigorously for 2 hours, then filtered through perlite and the cake is washed with water (10 mL). The aqueous solution of XVa can be used in the next oxidation step without any purification. Purity: 98.2 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 4.53 min
Column: RP-18 endcapped (5µm, 250 mm)
TLC: acetone - methanol (3:1) R_{f} = 0.13 (XVa)
Ethyl acetate - hexane (5:1) R_{f}= 0.10 (XIV)
Detection: UV, 1 % AgNO₃ in ethanolic solution

### Step 5. Production of Tetrabutylammonium 2α-methyl-2β-(1,2,3-triazol-1-yl)methylpenam-3α-carboxylate-1,1-dioxide (compound Xa)

To a cold solution of 0.306 g (1.00 mmol) ofXVa (as may be prepared from XV and NaHCO₃ or according to the alternate process described before) and 0.088 g (1.05 mmol) NaHCO₃, 0.373 g (1.05 mmol) tetrabutylammonium hydrogenpersulfate was added in small portions with vigorous stirring such that 5°C was not exceeded. The reaction mixture was stirred further 24 hours between 0-5°C and extracted with 5 x 10 ml of dichloromethane. The combined organic layers were washed with 5 ml of water and evaporated to dryness keeping the temperature of water bath below 20°C, yielding compound Xa.
Yield: 0.36 g (71 %)
Purity: 99.5 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.53 min
Column: RP-18 endcapped (5µm, 250 mm)

### Step 6. Production of Sodium 2α-methyl-2β-(1,2,3-triazol-1-yl)methylpenam-3α-carboxylate-1,1-dioxide (Tazobactam sodium)

As in the previous Examples, the residue containing compound Xa (0.40 g) was eluted with water on a column of Amberlite-H⁺ cation-exchange resin. The appropriate fractions were concentrated under reduced pressure and finally lyophilized, yielding Tazobactam sodium.
Yield: 0.20 g (85 %)
Purity: 99.5 % (HPLC)
HPLC mobile phase: 0.05 M KH₂PO₄ buffer, pH 2.3
Eluent A: 95 % of 0.05 M KH₂PO₄ buffer (pH 2.3) plus 5 % acetonitrile
Eluent B: 40 % of 0.05 M KH₂PO₄ buffer (pH 2.3) plus 60 % acetonitrile
Retention time: 11.53 min
Column: RP-18 endcapped (5µm, 250 mm)

## Claims

1. A process for the preparation of Tazobactam represented by formula (a) and/or pharmacological acceptable salts thereof 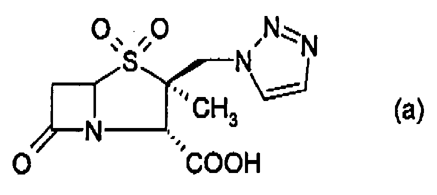 comprising the steps of
debrominating compound (k) 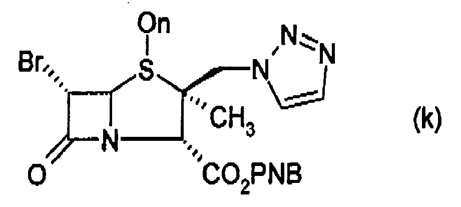 wherein PNB is para-Nitrobenzyl, n = 0,1 or 2 to give compound (1) 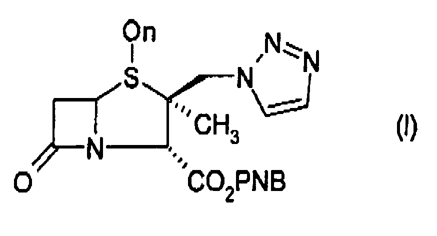 wherein n is as defined above;
deblocking compound (1) by catalytic hydrogenolysis or hydrolysis with a hydrogen sulfide salt to give compound (m) 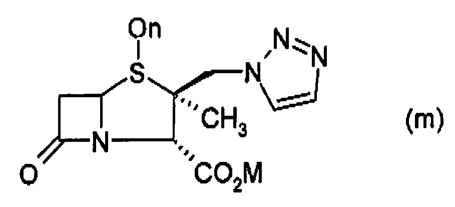 wherein n is as defined above and M is hydrogen or sodium;
reacting compound (m) with a quaternary amomnium salt NR₄Y wherein R is an substituted or unsubstituted alkyl chain, wherein in the case of n<2 that Y is an anion with oxidising capabilities, to yield compound (n), 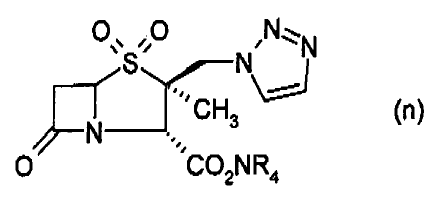 wherein R is defined above; and
converting compound (n) to a salt form and performing an ion exchange upon the NR₄ radical of the salt to yield compound (a) or a pharmacologically acceptable salt thereof.

2. A process for the deblocking of compound (o) or stereoisomers thereof by hydrolysis with a hydrogen sulphide salt 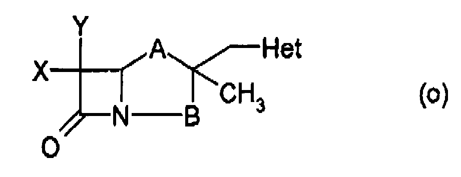 wherein X is halogen or hydrogen, Y is halogen or hydrogen, and Met is a substituted or unsubstituted heterocyclic ring, A is selected from the fragments: 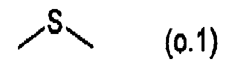 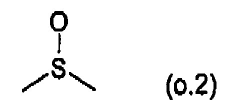 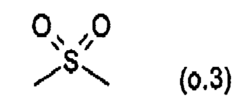 and B is the fragment: 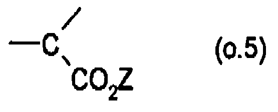 wherein Z is a substituted or unsubstitued benzylic protecting group.

3. The process of claim 2 **characterized in that** X and Y are hydrogen, A is the fragment o.1 and the hydrogensulphide salt is sodium hydrogen sulphide.

4. The process of claim 2 **characterized in that** X and Y are hydrogen, A is fragment 0.2 and the hydrogensulphide salt is sodium hydrogen sulphide.

5. A process for the preparation of Tazobactam by using compound (n) as an intermediate 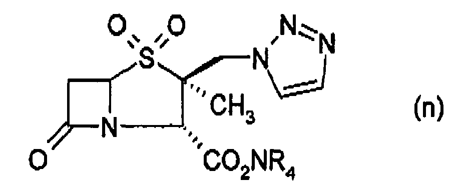 wherein R is an substituted or unsubstituted alkyl chain.

6. The process of claim 5 **characterized in that** R is butyl.

7. The process of claim 5 **characterized in that** the quaternary ammonium sulfonyl-compound (n), 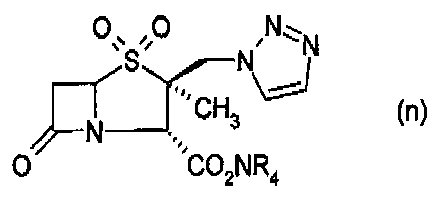 is formed from a thioether or sulfinyl-compound m, wherein n < 2, 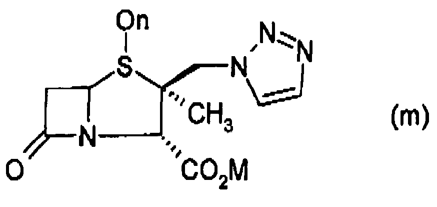 wherein M is hydrogen or sodium, by reaction with a quaternary ammonium salt possessing a counter-anion with oxidation capabilities.

8. Tetrabutylammonium 2a-methyl-2p-(1,2,3-triazol-1-yl)methylpenam-3a-carboxylate-1,1-dioxide (Xa)
